# FASCICULE DE BREVET EUROPEEN

(11) **EP 3 316 932 B1**
(45) Date de publication et mention de la délivrance du brevet: **24.04.2019**
(21) Numéro de dépôt: 16742361.5
(22) Date de dépôt: 30.06.2016
(51) Int. Cl.: A61M 5/20, A61M 5/30

(54) **DISPOSITIF D'INJECTION SANS AIGUILLE ÉQUIPÉ D'UN RESSORT DE COMPRESSION**
NADELLOSER INJEKTOR MIT DRUCKFEDER
NEEDLELESS INJECTOR WITH A COMPRESSION SPRING

(30) Priorité: 30.06.2015 FR 1556165
(43) Date de publication de la demande: 09.05.2018
(73) Titulaire: Crossject, 21000 Dijon (FR)
(72) Inventeur: VIVIEN, Gilles, 92240 Malakof (FR); CLOIX, Jean Christophe, 21600 Longvic (FR)
(74) Mandataire: Chevalier, Renaud Philippe
(86) Numéro de dépôt international: PCT/FR2016/051656
(87) Numéro de publication internationale: WO 2017/001795

(56) Documents cités:
- WO-A1-97/21457
- FR-A1- 2 815 544
- US-A1- 2014 060 263

## Description

L'invention concerne un dispositif d'injection sans aiguille qui est équipé d'un ressort de compression.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans un logement tubulaire délimité par le corps du dispositif, et qui est obturé par un piston amont et un piston aval entre lesquels est contenu le principe actif liquide.

L'extrémité libre aval, ou inférieure, du réservoir, coopère avec une buse d'injection qui délimite au moins un canal d'injection s'étendant axialement suivant un axe d'injection.

La buse d'injection est délimitée axialement par une face supérieure en appui axial sur le réservoir, et une face inférieure d'injection adaptée pour coopérer avec un opercule de fermeture.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appuie la buse d'injection sur sa peau.

Le déplacement du corps dans le capot permet le déclenchement du générateur de gaz, générant un gaz sous pression qui entraîne en déplacement les pistons pour injecter le principe actif à travers la peau de l'utilisateur, en passant par la buse d'injection.

Comme on peut le voir dans le document FR-A-2815544, un ressort de compression hélicoïdal est interposé axialement, suivant l'axe de coulissement, entre le corps et le capot, pour comprimer les tissus de la peau de l'utilisateur au cours de l'application de la buse sur la peau.

Le dispositif de percussion est déclenché lorsque le corps atteint sa position d'injection. A cet effet, l'utilisateur doit appuyer sur le capot du dispositif d'injection, pour faire coulisser le capot par rapport au corps du dispositif suivant une course de déclenchement déterminée.

Selon ce document FR-A-2815544, la course de déclenchement est donnée par la somme des espaces entre les spires du ressort. Pour augmenter cette course il faut augmenter cet espace et donc augmenter la hauteur total du dispositif.

De plus, pour augmenter l'effort de compression, il est nécessaire d'augmenter le diamètre de fil du ressort ou d'ajouter des spires, ce qui augemente la hauteur total du dispositif, notamment la hauteur du capot.

La présente invention vise notamment à résoudre ces inconvénients et se rapporte pour ce faire à un dispositif d'injection sans aiguille comportant :
- un capot,
- un système d'injection qui comprend au moins un piston, un réservoir de principe actif, une buse d'injection délimitant au moins un canal d'injection,
- un corps qui est enveloppé par le capot et qui est monté coulissant par rapport au capot, de bas en haut suivant un axe d'injection, entre une position de repos et une position d'injection,
- un ressort de compression qui est interposé axialement, suivant l'axe d'injection, entre le corps et le capot, pour comprimer les tissus de la peau de l'utilisateur au cours de l'application de la buse sur la peau,
- un générateur de gaz,
- un dispositif de percussion qui est conçu pour percuter le générateur de gaz, caractérisé en ce que le ressort de compression est un ressort hélicoïdal de forme tronconique qui s'étend suivant l'axe d'injection, et qui comporte une pluralité de spires conçues pour s'imbriquer axialement les unes dans les autres.

Ainsi, le ressort de compression selon l'invention permet de réduire l'espace vertical entre le corps et la face interne du capot à une épaisseur de spire du ressort, lorsque le corps occupe sa position de déclenchement.

De plus, le ressort de compression permet de faire varier le tarage du ressort en jouant sur la section du fil formant les spires, sans diminuer sensiblement la course de déclanchement du corps.

En outre, le ressort, de par sa forme tronconique et ses spires qui s'emboîtent sans jeu axial, l'ajout de spires n'impacte pas la course de déclenchement du corps.

Aussi, les spires du ressort ne sont pas en contact entre elles au cours du coulissement du corps, de sorte que le ressort n'émet pas de bruit de frottement.

Selon une autre caractéristique, le ressort de compression s'étend axialement depuis une spire haute qui est en appui sur le capot, jusqu'à une spire basse qui est en appui sur le corps, la spire haute présentant un diamètre supérieur au diamètre de la spire basse.

Le diamètre plus important de la spire haute du ressort permet une meilleure stabilité du ressort.

Selon une autre caractéristique, le capot délimite un logement qui reçoit la spire haute du ressort, pour bloquer en translation la spire haute latéralement.

Cette caractéristique permet également une meilleure stabilité du ressort.

De plus, le corps forme un bossage qui fait saillie verticalement vers le haut suivant l'axe d'injection, la spire basse du ressort étant montée autour du bossage, de sorte que la spire basse est bloquée latéralement.

Selon un mode de réalisation préféré, le ressort de compression est taré de façon à ce que l'effort nécessaire pour entraîner le corps depuis sa position de repos, jusqu'à sa position de déclenchement, est compris entre cinq et quarante cinq Newtons.

Enfin, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en perspective éclatée axialement, qui illustre un dispositif d'injection sans aiguille, selon l'invention ;
- la figure 2 est une vue simplifiée en section transversale, qui illustre le corps du dispositif de la figure 1 dans sa position de repos ;
- la figure 3 est une vue en section transversale similaire à celle de la figure 2, qui illustre le corps du dispositif de la figure 1 dans sa position de déclenchement.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

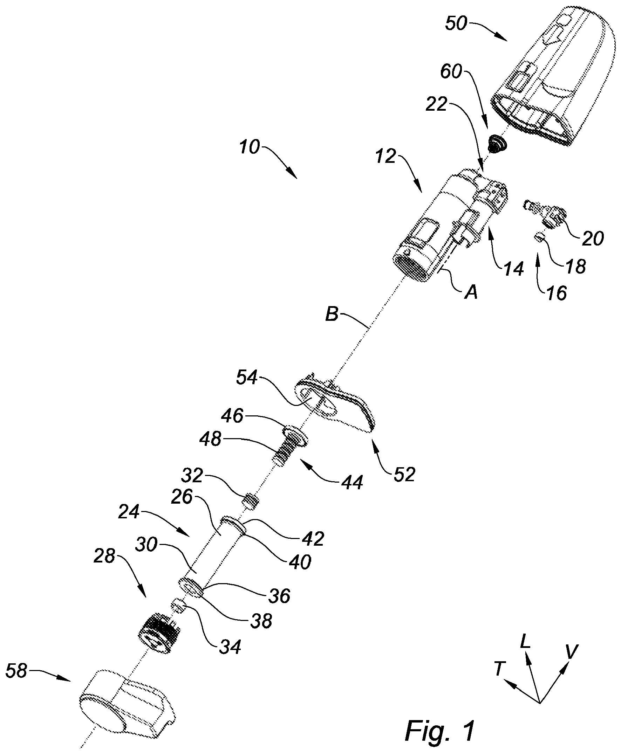

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

La buse 28 d'injection délimite trois canaux d'injection (non représentés) et est vissée sur une extrémité libre inférieure du corps 12.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston amont 32 et un piston aval 34 entre lesquels est contenu le principe actif 26, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

Aussi, selon la figure 1, une membrane 44 souple cylindrique comporte un siège annulaire 46 qui est interposé axialement entre la face supérieure 40 du réservoir 24 et l'orifice de sortie de la chambre d'expansion 22, et un corps 48 cylindrique qui s'étend axialement à l'intérieur du réservoir 24, au dessus du piston amont 32.

Le corps 48 de la membrane 44 est conçu pour s'étendre axialement, sous l'effet de la pression du gaz généré par le générateur de gaz 16, pour pousser le piston amont 32.

En référence à la figure 1, le corps 12 est enveloppé par un capot 50 creux qui délimite une ouverture inférieure fermée par une semelle 52 horizontale formant fond de capot.

La semelle 52 délimite un passage circulaire 54 autour de l'axe B d'injection qui est adapté pour le passage et le coulissement de la buse 28 d'injection et de l'extrémité aval du corps 12, de sorte que la buse 28 comporte un tronçon inférieur faisant saillie verticalement vers le bas hors du capot 50.

Aussi, le dispositif 10 d'injection est équipé d'un bouchon 58, représenté sur la figure 1, qui est monté sur le corps 12 de façon amovible par un moyen de verrouillage du type à baïonnette.

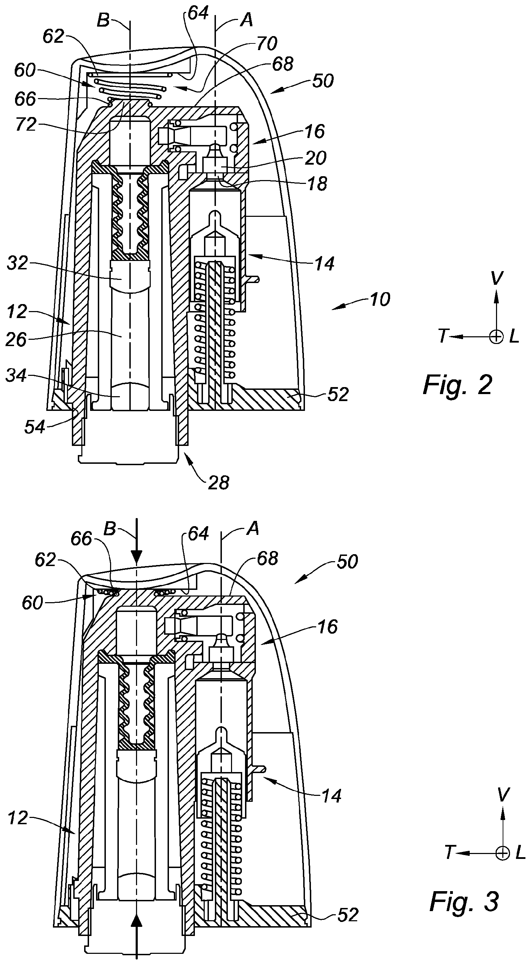

Comme on peut le voir aux figures 2 et 3, sur lesquelles le bouchon 58 n'est pas représenté, le corps 12 est monté coulissant par rapport au capot 50, de bas en haut suivant l'axe B d'injection, entre une position de repos illustrée à la figure 2, et une position d'injection illustrée à la figure 3.

Le déplacement du corps 12 à l'intérieur du capot 50 permet le déclenchement du générateur de gaz 16 qui génère un gaz sous pression entraînant en déplacement les pistons 32, 34 pour injecter le principe actif 26 à travers la peau de l'utilisateur, en passant par la buse 28 d'injection.

Aussi, le dispositif 10 d'injection comporte un ressort 60 de compression qui est interposé axialement, suivant l'axe B d'injection, entre le corps 12 et le capot 50, pour comprimer les tissus de la peau de l'utilisateur au cours de l'application de la buse 28 sur la peau, la buse 28 étant solidaire en déplacement du corps 12.

Le ressort 60 de compression est un ressort hélicoïdal de forme tronconique qui s'étend suivant l'axe B d'injection, et qui comporte une pluralité de spires, ici au nombre de quatre, qui sont conçues pour s'imbriquer axialement les unes dans les autres.

On entend par « ressort hélicoïdal de forme tronconique » un ressort dont les spires sont adaptées pour s'imbriquées axialement les unes dans les autres suivant l'axe central du ressort.

Plus particulièrement, le ressort 60 de compression s'étend axialement depuis une spire haute 62 qui est en appui sur une face interne 64 du capot 50, jusqu'à une spire basse 66 qui est en appui sur une face supérieure 68 du corps 12, la spire haute 62 présentant un diamètre supérieur au diamètre de la spire basse 66.

Le capot 50 délimite un logement 70 qui reçoit la spire haute 62 du ressort 60, pour bloquer en translation la spire haute 62 dans un plan horizontal.

De plus, la partie supérieure du corps 12 forme un bossage 72 qui fait saillie verticalement vers le haut suivant l'axe B d'injection, depuis la face supérieure 68 du corps 12.

La spire basse 66 du ressort 60 est montée autour du bossage 72, de sorte que la spire basse 66 est bloquée en translation dans un plan horizontal.

Suivant un exemple de réalisation préféré, le ressort 60 de compression est taré de façon à ce que l'effort nécessaire pour entraîner le corps 12 depuis sa position de repos, jusqu'à sa position de déclenchement, est compris entre cinq et quarante cinq Newtons.

Le ressort 60 de compression selon l'invention permet de réduire l'espace vertical entre le corps 12 et la face interne 64 du capot 50 à une épaisseur de spire du ressort 60, lorsque le corps 12 occupe sa position de déclenchement, comme on peut le voir à la figure 3.

Ainsi, le ressort 60 permet de faire varier le tarage du ressort en jouant sur la section du fil formant les spires, sans diminuer sensiblement la course de déclanchement du corps 12.

De plus, le ressort 60, de par sa forme tronconique et ses spires qui s'emboîtent sans jeu axial, l'ajout de spires n'impacte pas la course de déclenchement.

Aussi, les spires du ressort 60 ne sont pas en contact entre elles au cours du coulissement du corps 12, de sorte que le ressort 60 n'émet pas de bruit de frottement.

En outre, le diamètre plus important de la spire haute 62 du ressort 60 permet une meilleure stabilité du ressort 60.

## Revendications

1. Dispositif (10) d'injection sans aiguille comportant :
- un capot (50),
- un système d'injection qui comprend au moins un piston (32, 34), un réservoir (24) qui contient un principe actif (26), une buse (28) d'injection délimitant au moins un canal d'injection,
- un corps (12) qui est enveloppé par le capot (50) et qui est monté coulissant par rapport au capot (50), de bas en haut suivant un axe (B) d'injection, entre une position de repos et une position d'injection,
- un ressort (60) de compression qui est interposé axialement, suivant l'axe (B) d'injection, entre le corps (12) et le capot (50), pour comprimer les tissus de la peau de l'utilisateur au cours de l'application de la buse (28) sur la peau,
- un générateur de gaz (16),
- un dispositif (14) de percussion qui est conçu pour percuter le générateur de gaz (16),
**caractérisé en ce que** le ressort (60) de compression est un ressort hélicoïdal de forme tronconique qui s'étend suivant l'axe (B) d'injection, et qui comporte une pluralité de spires conçues pour s'imbriquer axialement les unes dans les autres.

2. Dispositif (10) d'injection sans aiguille selon la revendication 1, **caractérisé en ce que** le ressort (60) de compression s'étend axialement depuis une spire haute (62) qui est en appui sur le capot (50), jusqu'à une spire basse (66) qui est en appui sur le corps (12), la spire haute (62) présentant un diamètre supérieur au diamètre de la spire basse (66).

3. Dispositif (10) d'injection sans aiguille selon la revendication 2, **caractérisé en ce que** le capot (50) délimite un logement (70) qui reçoit la spire haute (62) du ressort (60), pour bloquer en translation la spire haute (62) latéralement.

4. Dispositif (10) d'injection sans aiguille selon la revendication 2, **caractérisé en ce que** le corps (12) forme un bossage (72) qui fait saillie verticalement vers le haut suivant l'axe B d'injection, la spire basse (66) du ressort (60) étant montée autour du bossage (72), de sorte que la spire basse (66) est bloquée latéralement.

5. Dispositif (10) d'injection sans aiguille selon l'une quelconque des revendications précédentes, **caractérisé en ce que** le ressort (60) de compression est taré de façon à ce que l'effort nécessaire pour entraîner le corps (12) depuis sa position de repos, jusqu'à sa position de déclenchement, est compris entre cinq et quarante cinq Newtons.

6. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadelloser Injektor (10), umfassend:
- eine Kappe (50),
- ein Injektionssystem, das mindestens einen Kolben (32, 34) und einen Behälter (24), der einen Wirkstoff (26) enthält, umfasst, wobei eine Injektionsdüse (28) mindestens einen Injektionskanal begrenzt,
- einen Körper (12), der von der Kappe (50) umgeben ist, und der gleitend mit Bezug auf die Kappe (50) von unten nach oben gemäß einer Injektionsachse (B) zwischen einer Ruheposition und einer Injektionsposition montiert ist,
- eine Druckfeder (60), die axial gemäß der Injektionsachse (B) zwischen dem Körper (12) und der Kappe (50) angebracht ist, um die Gewebe der Haut des Benutzers im Laufe der Anwendung der Düse (28) auf die Haut zu komprimieren,
- einen Gasgenerator (16),
- eine Schlagvorrichtung (14), die entworfen ist, um den Gasgenerator (16) zu schlagen,
**dadurch gekennzeichnet, dass** die Druckfeder (60) eine kegelstumpfförmige Schraubenfeder ist, die sich gemäß der Injektionsachse (B) erstreckt, und die eine Vielzahl von Windungen umfasst, die entworfen sind, um sich einander axial zu überlagern.

2. Nadelloser Injektor (10) nach Anspruch 1, **dadurch gekennzeichnet, dass** sich die Druckfeder (60) axial von einer oberen Windung (62), die auf der Kappe (50) aufliegt, bis zu einer unteren Windung (66) erstreckt, die auf dem Körper (12) aufliegt, wobei die obere Windung (62) einen größeren Durchmesser als den Durchmesser der unteren Windung (66) aufweist.

3. Nadelloser Injektor (10) nach Anspruch 2, **dadurch gekennzeichnet, dass** die Kappe (50) die Aufnahme (70) begrenzt, die die obere Windung (62) der Feder (60) empfängt, um in Translation die obere Windung (62) seitlich zu blockieren.

4. Nadelloser Injektor (10) nach Anspruch 2, **dadurch gekennzeichnet, dass** der Körper (12) eine Erhebung (72) bildet, die vertikal nach oben gemäß der Injektionsachse B vorspringt, wobei die untere Windung (66) der Feder (60) um die Erhebung (72) montiert ist, so dass die untere Windung (66) seitlich blockiert wird.

5. Nahtloser Injektor (10) nach einem der vorhergehenden Ansprüche, **dadurch gekennzeichnet, dass** die Druckfeder (60) derart tariert ist, dass die Kraft, die erforderlich ist, um den Körper (12) aus seiner Ruheposition in seine Auslöseposition anzutreiben, im Bereich zwischen fünf und fünfundvierzig Newton liegt.

6. Nahtloser Injektor (10) nach einem der vorhergehenden Ansprüche, **dadurch gekennzeichnet, dass** der Wirkstoff (26), der im Behälter (24) enthalten ist, ausgewählt ist aus der Gruppe, umfassend die folgenden Wirkstoffe:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazinhydrochlorid,
- Zuclopenthixolacetat,
- Danaparoid-Natrium,
- Enoxaparin-Natrium,
- Estradiolcypionat,
- Medroxyprogesteronacetat,
- Medroparin-Calcium,
- Methylprednisolonacetat
- Heparincalcium,
- Terbulin.

## Claims

1. A needleless injection device (10) including:
- a cover (50),
- an injection system which comprises at least one plunger (32,34), a reservoir (24) which contains an active ingredient (26), an injection nozzle (28) delimiting at least one injection channel,
- a body (12) which is wrapped by the cover (50) and which is slidably mounted relative to the cover (50), from bottom to top along an injection axis (B), between a rest position and an injection position,
- a compression spring (60) which is axially interposed, along the injection axis (B), between the body (12) and the cover (50), for compressing the tissues of the skin of the user during the application of the nozzle (28) on the skin,
- a gas generator (16),
- a striker device (14) which is designed to strike the gas generator (16),
**characterized in that** the compression spring (60) is a helical spring with a frustoconical shape which extends along the injection axis (B), and which comprises a plurality of coils designed to axially interlock into each other.

2. The needleless injection device (10) according to claim 1, **characterized in that** the compression spring (60) axially extends from a top coil (62) which bears on the cover (50), down to a bottom coil (66) which bears on the body (12), the top coil (62) presenting a diameter larger than the diameter of the bottom coil (66).

3. The needleless injection device (10) according to claim 2, **characterized in that** the cover (50) delimits a housing (70) which receives the top coil (62) of the spring (60), so as to laterally block in translation the top spire (62).

4. The needleless injection device (10) according to claim 2, **characterized in that** the body (12) forms a boss (72) which projects vertically upwards along the injection axis B, the bottom coil (66) of the spring (60) being mounted around the boss (72), so that the bottom coil (66) is laterally blocked.

5. The needleless injection device (10) according to any one of preceding claims, **characterized in that** the compression spring (60) is calibrated so that the force required to drive the body (12) from its rest position, up to its trigger position, is between five and forty-five Newtons.

6. The needleless injection device (10) according to any one of preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate,
- Heparin calcium,
- Terbuline.
